# EUROPEAN PATENT SPECIFICATION

(11) **EP 2 167 673 B1**
(45) Date of publication and mention of the grant of the patent: **05.01.2011**
(21) Application number: 08774233.4
(22) Date of filing: 23.06.2008
(51) Int. Cl.: C12P 13/02

(54) **PROCESS FOR THE CONVERSION OF C-TERMINAL PEPTIDE ESTERS OR ACIDS TO AMIDES EMPLOYING SUBTILISIN IN THE PRESENCE OF AMMONIUM SALTS**
VERFAHREN ZUR UMSETZUNG VON C-TERMINALEN PEPTIDESTERN ODER -SÄUREN ZU AMIDEN UNTER VERWENDUNG VON SUBTILISIN IN GEGENWART VON AMMONIUMSALZEN
PROCÉDÉ DE CONVERSION D'ESTERS OU D'ACIDES C-TERMINAUX DE PEPTIDES EN AMIDES À L'AIDE DE LA SUBTILISINE EN PRÉSENCE DE SELS D'AMMONIUM

(30) Priority: 25.06.2007 EP 07012410
(43) Date of publication of application: 31.03.2010
(73) Proprietor: N.V. Organon, 5349 AB Oss (NL)
(72) Inventor: EGGEN, Ivo, Franci, NL-5340 BH Oss (NL); BOERIU, Carmen, G., NL-6703 EV Wageningen (NL)
(74) Representative: Quelle-Fontijn, Monique
(86) International application number: PCT/EP2008/057972
(87) International publication number: WO 2009/000814

(56) References cited:
- EP-A- 1 291 356
- CHEN, S.T. ET AL.: "Facile Amide Bond Formation From Esters of Amino Acids and Peptides Catalyzed by Alkaline Protease in Anhydrous tert-Butyl Alcohol Using Ammonium Chloride/Triethylamine as a Source of Nucleophilic Ammonia" SYNTHESIS, vol. 09, 1993, pages 858-860, XP002458658 cited in the application
- KLEIN, J.U. ET AL.: "The Applicability of Subtilisin Carlsberg in Peptide Synthesis" JOURNAL OF PEPTIDE SCIENCE, vol. 6, no. 11, 13 November 2000 (2000-11-13), pages 541-549, XP002401403

## Description

The invention relates to a process for selective enzymatic amidation of C-terminal esters or acids of peptide substrates in solution-phase synthesis of peptides.

Many biologically active peptides contain a C-terminal primary amide function, *e.g*. gonadorelin, oxytocin, and arginine vasopressin. There are several strategies for the synthesis of peptide amides in solution, which can be roughly divided into four different categories.

In the most straightforward approach, synthesis is started from the free C-terminal amino acyl amide, maintaining the amide in its free form during the entire synthesis. Although the most obvious approach on first examination, syntheses in solution in the presence of a free amide function on the growing peptide chain are often fraught with serious solubility problems due to hydrogen bonding. Moreover, for peptide synthesis on a manufacturing scale, the DioRaSSP® method is particularly preferred (EP-A-1,291,356; US 6,864,357; Eggen I. et al., Org. Process Res. Dev. 2005, 9, 98 -101; Eggen I. et al., J. Peptide Sci., 2005, 11, 633-641). In this method, the growing peptide chain is more or less anchored in a permanent organic phase. However, when using a free amide function, there is a significant risk of losing a substantial portion of the growing peptide during aqueous washings, especially in the earlier cycles of the synthesis.

In order to circumvent the aforementioned solubility problems, the amide function may be protected during the synthesis, hence starting from the C-terminally protected amino acyl amide. This approach also ensures the anchoring of the growing peptide in the organic phase. Protecting groups for the amide function may be selected from benzyl (Weygand, F. et al., Chem. Ber., 1968, 101, 3623-3641), benzhydryl (Sakakibara, S. et al., Bull. Chem. Soc. Jpn., 1967, 40, 2164-2167), triphenylmethyl (Sieber, P. et al., B., Tetrahedron Lett., 1991, 32, 739-742), xanthenyl (Shimonishi, Y. et al., Bull. Chem. Soc. Jpn., 1962, 35, 1966-1970), and cyclopropylmethyl type moieties (Carpino, L.A. et al., J. Org. Chem., 1995, 60, 7718-7719), whose lability towards acid is determined by the substituents attached to this moiety. A general problem is the poor accessibility and commercial availability of the starting amino acid derivatives, especially if these contain protected side chains. Moreover, cleavage of the protecting group from the amide function under acidic conditions may result in the formation of alkylated analogues, depending on the actual sequence of the peptide, the conditions for deprotection and the nature of the protecting group.

A third option for the synthesis towards peptides with a C-terminal amide function is to start from amino acyl esters, which allow selective cleavage towards the free carboxylic function in the presence of acid-labile protecting groups on the side chains of the peptide. The free carboxylic function is subsequently activated to allow reaction with ammonia c.q. ammonium. Esters within the scope of this approach include 9-fluorenylmethyl (Fm) (Cunningham, B.R. et al., Tetrahedron Lett., 1994, 35, 9517-9520) and 2-(4-nitrophenylsulfonyl)ethyl (Nse) esters (Carreño, C. et al., J. Peptide Res., 2000, 56, 63-69) which are cleaved under basic conditions, 2-(trimethylsilyl)ethyl *(*Tmse) esters which are cleaved by fluoridolysis (Sieber, P., Helv. Chim. Acta, 1977, 60, 2711-2716), and 2,4-dimethoxybenzyl (Dmb) esters which are cleaved by mild acidolysis (McMurray, J.S., Tetrahedron Lett., 1991, 32, 7679-7682). The main problem with this approach is that it comprises two potential racemization-inducing steps of the C-terminal amino acid derivative, *i.e*. the esterification step and the actual amidation step. Moreover, the cleavage step may lead to side reactions within the peptide sequence, such as conversion of internal Asp(OBu*^{t}*) residues to the succinimide under basic (Mergler, M. et al., J. Pept. Sci., 2003, 9, 36-46) or fluoridolytic conditions. Finally, the ester function itself may not be completely stable during the assembly of the peptide sequence; *e.g*., benzyl-type esters (Fm and Dmb) are cleaved during hydrogenolysis, thus ruling out the use of the benzyloxycarbonyl (Z) group for temporary protection of the amino function.

The last option for the synthesis towards peptides with a C-terminal amide function is to start from simple primary amino acyl esters, such as methyl, ethyl or benzyl esters, which are at a later stage converted to the amides *via* direct ammonolysis. These are in general widely available and easily accessible starting compounds. However, this approach is limited by the fact that the ammonolytic conditions also induce side reactions within the peptide sequence, such as racemization and conversion of internal Asp(OBu*^{t}*) residues to the succinimide.

It may be concluded that the use of simple primary amino acyl esters as the starting materials in the synthesis towards peptides with a C-terminal amide function is preferred, if the chemical ammonolysis can be replaced by a mild selective method for the amidation.

Enzymatic methods have gained interest in peptide synthesis during the past couple of years. Enzymes often show high chemo-, regio- and stereoselectivity. Furthermore, enzymes usually operate under very mild conditions, at neutral pH values and at temperatures of 20-50°C. Thus, under such conditions, side reactions can be circumvented.

In mammals several amidating enzymes are expressed, including bifunctional Peptidylglycine α-Amidating Monooxygenase (PAM) and the monofunctional enzymes Peptidyl α-Hydroxylating Monooxygenase (PHM) and Peptidyl Amidoglycolate Lyase (PAL) (Kulathila, R. et al., Nat. Prod. Rep., 1999, 16, 145-154). These enzymes need a glycine at the C-terminus and are therefore not generally applicable. Furthermore, these isolated enzymes are very costly and have found no application in the laboratory ( 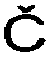 e 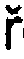 ovský, V. *et al.,* Biotechnol. Appl. Biochem., 2001, 33, 183-187).

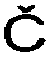 e 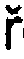 ovský *et al.* reported ( 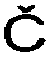 e 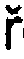 ovský, V. *et al.,* Angew. Chem. Int. Ed., 1998, 37, 1885-1887) the application of an enzyme isolated from orange peel in the amidation of the free acid of a C-terminal peptide. Despite some optimization yields never exceeded 35%. Furthermore, in another publication the yields seemed to be largely dependant on the substrate used ( 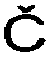 e 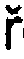 ovský, V., Kula, M.R., Biotechnol. Appl. Biochem., 2001, 33, 183-187). Finally, the 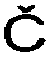 e 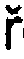 ovský method is based on conversion of peptide with a free C-terminal acid to the corresponding amide. Since solution-phase synthesis usually yields a C-terminal ester, an additional synthesis step is required to deprotect the ester function. Accordingly, it will be very difficult to apply this orange peel enzyme in an industrial process.

The use of lipases has shown a wide spread application in organic chemistry. Several publications report the use of *Candida antarctica* lipase in the amidation of organic compounds, resulting in substituted and non-substituted amides (Reyes-Duarte, D. et al., Biotech Lett., 2002, 24, 2057-2061; Sánchez, V.M. et al., J. Org. Chem. 1999, 64, 1464-1470; Torre, O. et al., Adv. Synth. Catal. 2005, 347, 1007-1014; Maugard, T. et al., Tetrahedron, 1997, 53, 14, 5185-5194; Zoete de, M.C. et al., Sheldon, R.A. Journal Molecular Catalysis B: Enzymatic, 1996, 1, 109-113; Litjens, M.J.J. et al., Tetrahedron, 1999, 55, 12411-12418). The application of this enzyme in peptide chemistry however remains to be reported.

Finally, Chen S-T. et al., Synthesis, 1993, 858 -860, reports the enzymatic amidation of esters of amino acids and C-terminal peptide esters with Alcalase (free subtilisin) in the presence of ammonium chloride / triethyl amine at pH 10.6 and higher. Reported yields are between 50 and 70%. Chen reports in a table the amidation of a dipeptide with a 68% conversion in 12 hours. The amidation of a tripeptide is also reported. However, the conversion is not listed. It may also be mentioned here that it is highly undesired in solution-phase synthesis of peptide to use such high pH as this might result in the destruction of the peptide. Therefore, mild conditions, *i.e*. a pH of 10 or lower, are preferred.

Therefore, despite these positive developments enzymatic amidation of the C-terminal ester of a peptide by means of a commercially available enzyme with high yields remains a daunting challenge hitherto.

A new process has now been found for the amidation of C-terminal esters or acids of peptide substrates in solution-phase synthesis of peptides, comprising amidating one or more peptide substrates comprising C-terminal esters or acids using the protease subtilisin in any suitable form in the presence of an ammonium salt derived from an acid having a pKa above 0.

Surprisingly, it has been found that with the process of the present invention high yields of the amidated product can be obtained, while endopeptidase activity is substantially suppressed.

With C-terminal acids of peptide substrates is meant the free C-terminal acid of a peptide.

Preferred are the C-terminal esters of peptide substrates. Since solution-phase synthesis usually yields a C-terminal ester, an additional synthesis step is required to deprotect the ester function. Furthermore, the amidation of the free C-terminal acid of a peptide is much slower than the amidation of the C-terminal ester.

The esters of the C-terminal esters of the peptide substrate may be selected from the group of C₁₋₁₂ (ar)alkyl esters, *e.g*. methyl, ethyl, propyl, butyl, and benzyl esters, in particular primary C₁₋₁₂ (ar)alkyl esters, preferably primary C₁₋₄ alkyl esters, more preferably ethyl and methyl esters. The C-terminal methyl ester of the peptide substrate is the most preferred embodiment.

The ammonium salt is derived from an acid having a pKa above 0, preferably from an acid having a pKa above 3.5, most preferably, having a pKa between 3.5 and 7. Examples include diammonium hydrogen phosphate, ammonium dihydrogen phosphate, ammonium fluoride, or ammonium sulphite hydrate.

Alternatively, the ammonium salt may have the following chemical structure (I): 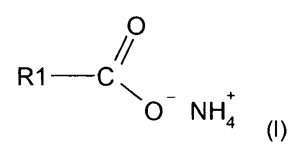 wherein
R1 is selected from the group of hydrogen, C₁₋₁₂ (ar)alkyl, C₆₋₁₂ aryl, -N(R2)₂, -OH, and R3-O⁻ NH₄⁺,
R2 is selected from the group of hydrogen and/or C₁₋₄ alkyl, and
R3 is a bond, a carbonyl group, or a C₁₋₄ alkyl carbonyl group, optionally substituted with one or more hydroxyl groups and/ or -COO⁻ NH₄⁺,
optionally in hydrated form.

Examples of ammonium salts according to the above-mentioned formula include ammonium carbamate, ammonium acetate, ammonium tartrate, ammonium benzoate, ammonium citrate, ammonium formate, ammonium oxalate monohydrate, ammonium carbonate, ammonium bicarbonate, and mixtures thereof.

Preferably, R1 is selected from the group of C₁₋₁₂ (ar)alkyl, C₆₋₁₂ aryl, -NH₂, -OH, and -O⁻ NH₄⁺.

Preferred embodiments are selected from ammonium carbamate, ammonium carbonate, ammonium bicarbonate, ammonium acetate, ammonium benzoate, and mixtures thereof.

Ammonium carbamate is the most preferred embodiment.

It is understood that ammonium carbamate cannot be derived in practice from carbamic acid in view of the fact that carbamic acid as such is an unstable compound and thus therefore does not exist. However, for the sake of the present invention it is defined that ammonium carbamate is an ammonium salt derived from carbamic acid, having a pKa between 4.2 and 7 (Masuda K. et al., Tetrahedron, 2005, 61, 213-229).

The protease subtilisin (EC 3.4.21.62) may be used in the process of the invention in any form, thus it may be used in soluble and/or crystallized form, but also in immobilized form or other insoluble form, *e.g*. in the form of cross-linked enzyme aggregates (CLEA) or cross-linked enzyme crystals (CLEC).

The new process of this invention may conveniently be used in the production of protected or unprotected peptides.

Preferably, the C-terminal ester or acid of the peptide substrates comprises a C-terminal acyl residue which is an α-amino acyl residue from natural or synthetic origin. The C-terminal α-amino acyl residue may be protected or unprotected at the side chain. In particular preferred are C-terminal α-amino acyl residues selected from Ala, protected Cys, protected Asp, protected Glu, Phe, Gly, His, (protected) Lys, Leu, Met, Asn, Gln, (protected) Arg, (protected) Ser, Thr, Val, (protected) Trp and (protected) Tyr, wherein the brackets around the word "protected" mean that the residue can be present in both side-chain protected and unprotected form. The three-letter code for amino acids is used here according to IUPAC nomenclature (IUPAC-IUB Commission (1985) J. Biol. Chem. 260, 14-42).

In a further preferred embodiment, protected or unprotected peptide substrates used in the process of this invention are prepared according to DioRaSSP®. Thus, a peptide substrate comprising a C-terminal ester or acid is preferably prepared according to this process for rapid solution synthesis of a peptide in an organic solvent or a mixture of organic solvents, the process comprising repetitive cycles of steps (a)-(d):
(a) a coupling step, using an excess of an activated carboxylic component to acylate an amino component,
(b) a quenching step in which a scavenger is used to remove residual activated carboxylic functions, wherein the scavenger may also be used for deprotection of the growing peptide,
(c) one or more aqueous extractions and
optionally, (d) a separate deprotection step, followed by one or more aqueous extractions,
whereby in at least one cycle in process step b an amine comprising a free anion or a latent anion is used as a scavenger of residual activated carboxylic functions. The amine is preferably benzyl β-alaninate or a salt thereof.

The molar ratio of ammonium salt to peptide substrate may range from 2:1 to 20:1, preferably 5:1 to 12:1, more preferably 6:1 to 10:1.

The amidation of the process of the present invention may be performed in one or more organic solvents. Polar organic solvents are preferred, and in particular the organic solvent is selected from *N*,*N*-dimethylformamide (DMF), *N*-methyl-2-pyrrolidone (NMP), dioxane, *N*,*N*-dimethylacetamide (DMA), dichloromethane (DCM), tetrahydrofuran (THF), acetonitrile, *tert*-butanol, *tert*-amyl alcohol, dichloroethane (DCE), *tert*-butyl methyl ether (MTBE), and mixtures thereof. Preferred are mixtures of *tert*-butanol and DMF, *tert*-butanol and NMP, *tert*-amyl alcohol and DMF, or *tert*-amyl alcohol and NMP.

These mixtures may be used in a ratio of 40:60 to 95:5 (v/v), preferably 60:40 to 90:10 (v/v), more preferably in a ratio of 82.5:17.5 (v/v).

Preferably, a small amount of water is present in the reaction mixture. It has been found that in a fully anhydrous "dry" system the enzyme is inactivated due to lack of water. However, when the water concentration is too high in the reaction mixture undesired ester hydrolysis occurs to a significant extent (*i.e*. formation of a C-terminal free acid) at the cost of amidation. Accordingly, the percentage of water in the mixture may range from 0.0001 to 5% (v/v), preferably from 0.01 to 2% (v/v). It is especially useful when the percentage of water in the mixture is about 0.1 to 1 % (v/v).

Water may be added on purpose or by introducing water-containing reagents. For example, when cross-linked enzyme aggregates (CLEA's) are used, water is introduced automatically in the reaction mixture due to its presence in CLEA's. Introduction of water in the reaction mixture may be carried out in the form of an aqueous buffer. Suitable buffers may be selected from buffers which are generally used for transformations using proteolytic enzymes. In particular, the aqueous buffer is a phosphate, borate or 2-amino-2-hydroxymethyl-1,3-propanediol (TRIS) buffer.

The pH at which the reaction is performed may be selected from the range of 5.5-10, preferably 5.5-8.5, more preferably 6-8, and most preferably the pH is 7.

Reaction temperatures for the amidation may suitably be selected in the range of 15-60°C, in particular 20 to 40°C. Preferred is a reaction temperature of 30°C.

The amount of enzyme may suitably be selected in the range of 1 to 50 wt.% of enzyme related to the peptide substrate, preferably 5 to 10 wt.%.

In a further embodiment of the invention, the amidation is performed by stepwise adding portions of the protease subtilisin (in any suitable form) to the reaction mixture comprising one or more peptide substrates comprising C-terminal esters or acids.

In an alternative embodiment of the invention, the amidation is performed by stepwise adding portions of the ammonium salt to the reaction mixture comprising one or more peptide substrates comprising C-terminal esters or acids.

The peptide substrates, of which the C-terminal esters or acids are amidated in the process of the invention, may carry protecting groups in other parts of their peptide sequence.

The amidation can be carried out with one peptide substrate as well as a mixture thereof. Such a mixture may comprise peptide substrates having only C-terminal esters, peptide substrates having only free C-terminal acids, or peptide substrates having both C-terminal esters and free C-terminal acids.

A suitable process according to the present invention is as follows.

To a solution of a C-terminal ester or acid of a peptide substrate in a suitable organic solvent (or mixture of organic solvents) ammonium salt is added. The reaction mixture is incubated above ambient and the reaction is started by the addition of an enzyme catalyst. The enzyme can be added in solution (e.g. Subtilisin A in phosphate buffer), in suspension or as solid material (e.g. native enzyme, immobilised enzyme or cross-linked enzyme). When substrate conversion has reached a desired level, *e.g*. higher than 90%, the peptide may be isolated according to the knowledge of the skilled person.

The term 'substrate' herein means an entity which is converted to a product by the protease subtilisin in any form. This product may be a final peptide product whereby if present only the protected functional side chains have still to be deprotected. Alternatively, this product may also be a peptide fragment which subsequently is reacted with other peptide fragments in a convergent synthesis to obtain a longer peptide with the required final number of amino acids.

A person skilled in the art can easily identify suitable substrates, for instance by performing a simple test amidation of a selected peptide comprising a C-terminal ester functionality or a free C-terminal acid under suitable conditions as described herein before and following conversion *e.g*. by HPLC techniques.

It is established that the process is very suitable to prepare amides of short peptides comprising up to 5 amino acids. Furthermore, a skilled person will also be able to prepare longer peptides. Peptides may also be prepared with D-amino acids.

The term 'protected' means that the functional groups (within the peptide) are protected with suitable protecting groups. A person skilled in the art will know which type of protection to select for which type of functional group. For example, amine functions present in the compounds may be protected during the synthetic procedure by an N-protecting group, which means a group commonly used in peptide chemistry for the protection of an α-amino group, like the *tert*-butyloxycarbonyl (Boc) group, the benzyloxycarbonyl (Z) group, or the 9-fluorenylmethyloxycarbonyl (Fmoc) group.

Overviews of amino protecting groups and methods for their removal is given in Geiger R. and König W. (1981) in Peptides: Analysis, Synthesis, Biology, Vol 3, Gross E. and Meienhofer, J., eds, Academic Press, New York, pp. 1 -99, and Peptides: Chemistry and Biology, Sewald N. and Jakubke H.-D., eds, Wiley-VCH, Weinheim, 2002, pp. 143 - 154. Functions of the *tert*-butyl type or functions of similar lability are preferred for the protection of other functional groups on the side chains; these include - but are not limited to - *tert*-butyl (Bu*^{t}*) for the protection of the Asp, Glu, Ser, Thr and Tyr side chains, *tert*-butoxycarbonyl (Boc) for the protection of the Lys and Trp side chains, trityl (Trt) for the protection of the Asn, Gln and His side chains and 2,2,5,7,8-pentamethylchromane-6-sulfonyl (Pmc) or 2,2,4,6,7-pentamethyldihydrobenzofurane-5-sulfonyl (Pbf) for the protection of the Arg side chain [Barany, G. and Merrifield, R.B. (1980) in: 'The Peptides', vol. 2 (Gross, E. and Meienhofer, J., eds.) Academic Press, New York, pp. 1-284; for Trp(Boc): Franzén, H. et al. (1984) J. Chem. Soc., Chem. Commun., 1699-1700; for Asn(Trt) and Gln(Trt): Sieber, P. et al. (1991) Tetrahedron Lett. 32, 739-742; for His(Trt): Sieber, P. et al. (1987) Tetrahedron Lett. 28, 6031-6034; for Pmc: Ramage, R. et al. (1987) Tetrahedron Lett. 28, 2287-2290; for Pbf: Carpino, L.A. et al. (1993) Tetrahedron Lett. 34, 7829-7832].

The invention is further illustrated by the following examples.

### EXAMPLES

The peptides have been produced according to conventional solution phase methods for peptide synthesis.

The free enzyme Subtilisin A was purchased from Novozymes unless otherwise stated. Alcalase CLEA, Savinase CLEA, and CAL-B CLEA were obtained from CLEA Technologies B.V., Delft, The Netherlands. All other enzymes were purchased from Sigma Aldrich.
- P-NH₂: = Z-peptide-NH₂, e.g. Z-Val-Phe-NH₂
- P-OH: = Z- peptide-OH, e.g. Z-Val-Phe-OH
- P-OMe: = Z- peptide-OMe, e.g. Z-Val-Phe-OMe

- Bu*^{t}*OH: = *tert*-butanol
- Am*^{t}*OH: = *tert*-amyl alcohol
- DMF: = *N*,*N*-dimethylformamide
- NMP: = *N*-methyl-2-pyrrolidone
- DMA: = *N*,*N*-dimethylacetamide
- DCM: = dichloromethane
- THF: = tetrahydrofuran
- DCE: = dichloroethane

### Example 1 and Comparative Examples A - G

### General procedure for screening the effect of enzyme:

A stock solution of Z-Ala-Phe-OMe in DMF (200 mM) was prepared. 0.5 mmol of ammonium carbamate was added to 0.25 ml of the dipeptide stock solution. Next, 0.625 ml DMF and 4.125 ml Bu*^{t}*OH was added. Depending on the enzyme used 10 µl phosphate buffer 0.1 M pH 7.0 was added. The reaction mixture was incubated at 30°C and the reaction was started by the addition of approximately 20 mg of enzyme. A sample (2 ml) was taken after two hours. 2 ml of acetonitrile was added and the mixture was centrifuged. The supernatants were analyzed by HPLC. The results are shown in Table 1.

**Table 1**

| **Ex.** | **H₂O/Bu*^{t}*OH/ DMF** | **Z-Ala-Phe-NH₂ a/a%** | **Z-Ala-Phe-OH a/a%** | **Z-Ala-Phe-OMe a/a%** | **Enzyme** |
|---|---|---|---|---|---|
| 1 | 0.2/82.5/17.5 | 86.2 | 13.4 | 0.4 | Alcalase CLEA |
| A | 0.2/82.5/17.5 | 5 | 2.6 | 92.4 | Savinase CLEA |
| B | 0.2/82.5/17.5 | 0 | 0 | 100 | CAL-B CLEA |

As is seen from the results in Table 1 *Candida Antartica* Lipase -B CLEA (CAL-B CLEA) and Savinase-CLEA are not active.

In similar protocols as described above Carboxypeptidase A (Comp. Ex. C), Lipase CC (*Candida Cylindracea*) (Comp. Ex. D), Lipase CR (*Candida Rugosa*) (Comp. Ex. E), Esterase HL (hog liver) (Comp. Ex. F), and Lipase CA (AR) (CAL-B immobilized on acrylic resin) (Comp. Ex. G) were not active. The reaction mixtures were also analyzed after 16 hours, providing similar results.

### Examples 2 and 3 and Comparative Example H

### General procedure for screening the effect of ammonium source

A stock solution of Z-Ala-Phe-OMe in DMF (50 mM) was prepared. 0.5 mmol of the ammonium source was added to 1 ml of the dipeptide stock solution. 4 ml Bu*^{t}*OH was added. 50 µl phosphate buffer 0.1 M pH 7.6 was added. The reaction mixture was incubated at 40°C and the reaction was started by the addition of 5 mg of Alcalase-CLEA. A sample (2 ml) was taken after two hours. 2 ml of acetonitrile was added and the mixture was centrifuged. The supernatants were analyzed by HPLC. The results are shown in Table 2.

**Table 2**

| **Ex.** | **Ammonium source** | **Composition reaction mixture** | | |
|---|---|---|---|---|
| | | **Z-Ala-Phe-NH₂ a/a%** | **Z-Ala-Phe-OH a/a%** | **Z-Ala-Phe-OMe a/a%** |
| 2 | (NH₄)₂CO₃ | 11.8 | 6.8 | 81.3 |
| 3 | NH₂CO₂NH₄ | 16.1 | 8.7 | 75.2 |
| H | NH₄Cl | 0.4 | 7.2 | 92.4 |

### Example 4

### Procedure for enzymatic amidation of a tripeptide:

A stock solution of Z-Ala-Phe-Ala-OMe in DMF (200 mM) was prepared. 0.5 mmol of NH₂CO₂NH₄ was added to 0.25 ml of the tripeptide stock solution. 0.625 ml DMF and 4.125 ml Bu*^{t}*OH was added. The reaction mixture was incubated at 30°C and the reaction was started by the addition of 20 mg of Alcalase-CLEA. A sample (2 ml) was taken after two hours and after four hours. 2 ml of acetonitrile was added and the mixture was centrifuged. The supernatants were analyzed by HPLC. The results are presented in Table 3.

**Table 3**

| **Time (hours)** | **Z-Ala-Phe-Ala-NH₂ a/a%** | **Z-Ala-Phe-Ala-OH a/a%** | **Z-Ala-Phe-Ala-OMe a/a%** |
|---|---|---|---|
| 2 | 69.7 | 10.2 | 18.3 |
| 4 | 79.0 | 10.6 | 8.9 |

### Example 5

### Effect of "dry" environment on the amidation of a dipeptide with free subtilisin

All solvents were dried with mol sieves prior to the experiment.

A stock solution of Z-Ala-Phe-OMe in DMF (200 mM) was prepared. 0.5 mmol of ammonium carbamate was added to 0.25 ml of the dipeptide stock solution. 0.625 ml DMF and 4.125 ml Bu*^{t}*OH was added. The reaction mixture was incubated at 30°C and the reaction was started by the addition of 1 mg of enzyme Subtilisin A. A sample (2 ml) was taken after two hours. 2 ml of acetonitrile was added and the mixture was centrifuged. The supernatants were analyzed by HPLC. The results are shown in Table 4.

**Table 4**

| **Water/Bu*^{t}*OH/DMF** | **Z-Ala-Phe-NH₂ a/a%** | **Z-Ala-Phe-OH a/a%** | **Z-Ala-Phe-OMe a/a%** |
|---|---|---|---|
| 0/82.5/17.5 | 1.7 | 0.4 | 97.9 |

### Examples 6 to 14

### Effect of additional ammonium carbamate and/or enzyme on the amidation of a dipeptide when Subtilisin A is used

A stock solution of Z-Ala-Phe-OMe in DMF (200 mM) was prepared. 0.5 mmol of ammonium carbamate was added to 0.25 ml of the dipeptide stock solution. DMF and Bu*^{t}*OH was added corresponding to the ratio given in Table 5, for a total reaction volume of 5 ml. The reaction mixture was incubated at 30°C and the reaction was started by the addition of 10 µl of enzyme solution (20 mg Subtilisin A in 200 µl phosphate buffer 0.1 M pH 7.0) (Initial conditions: 10 mM Z-Ala-Phe-OMe, 100 mM NH₂COONH₄, pH 7, 30°C). A sample (2 ml) was taken after 1.5 hours for experiments 6, 9, and 12, and after 3 hours for experiments 7, 8, 10, 11, 13, and 14. 2 ml of acetonitrile was added and the mixture was centrifuged. The supernatants were analyzed by HPLC. The results are shown in Table 5.

**Table 5**

| **Ex.** | **H₂O/Bu*^{t}*OH/DMF** | **Z-Ala-Phe-NH₂ a/a%** | **Z-Ala-Phe-OH a/a%** | **Z-Ala-Phe-OMe a/a%** | **Comments** |
|---|---|---|---|---|---|
| 6 | 0.2/82.5/17.5 | 62.1 | 11.6 | 26.2 | |
| 7 | 0.2/82.5/17.5 | 75 | 10.7 | 14.2 | extra carbamate (0.5 mmol at 1.5h) |
| 8 | 0.2/82.5/17.5 | 77.2 | 12.3 | 10.6 | extra carbamate (0.5 mmol) & enzyme (10 µl) at 1.5 h |
| 9 | 0.2/70/30 | 67.3 | 9.6 | 23.1 | |
| 10 | 0.2/70/30 | 75.8 | 9.3 | 14.9 | extra carbamate (0.5 mmol at 1.5h) |
| 11 | 0.2/70/30 | 81.7 | 10.9 | 7.5 | extra carbamate (0.5 mmol) & enzyme (10 µl) at 1.5 h |
| 12 | 0.2/40/60 | 53.6 | 7.2 | 39.1 | |
| 13 | 0.2/40/60 | 56.5 | 6.3 | 37.2 | extra carbamate (0.5 mmol at 1.5h) |
| 14 | 0.2/40/60 | 68.6 | 8.4 | 23 | extra carbamate (0.5 mmol) & enzyme (10 µl) at 1.5 h |

The results show that addition of extra ammonium carbamate and/ or enzyme results in an increase in substrate conversion and formation of higher amounts of amide.

### Examples 15 to 18

### Effect of additional ammonium carbamate on the amidation of a dipeptide when Alcalase CLEA is used

A stock solution of Z-Ala-Phe-OMe in DMF (200 mM) was prepared. 0.5 mmol of the ammonium source was added to 0.25 ml of the dipeptide stock solution. 0.625 ml DMF and 4.125 ml Bu*^{t}*OH was added. 10 µl phosphate buffer 0.1 M pH 7.0 was added in experiments 17 and 18. The reaction mixture was incubated at 30°C and the reaction was started by the addition of approximately 20 mg of Alcalase-CLEA. A sample (2 ml) was taken after two hours. 2 ml of acetonitrile was added and the mixture was centrifuged. The supernatants were analyzed by HPLC. The results are shown in Table 6 (initial conditions: 10 mM Z-Ala-Phe-OMe, 100 mM NH₂COONH₄, pH 7, 30°C).

**Table 6**

| **Ex.** | **H₂O/Bu*^{t}*OH/DMF** | **Z-Ala-Phe-NH₂ a/a%** | **Z-Ala-Phe-OH a/a%** | **Z-Ala-Phe-OMe a/a%** | **Comments** |
|---|---|---|---|---|---|
| 15 | 0/82.5/17.5 | 90.9 | 8.4 | 0.7 | |
| 16 | 0/82.5/17.5 | 91.1 | 7.8 | 1.1 | extra carbamate (0.05 mmol at 1 h) |
| 17 | 0.2/82.5/17.5 | 84.7 | 14.4 | 0.9 | |
| 18 | 0.2/82.5/17.5 | 78.9 | 20.1 | 1 | extra carbamate (0.05 mmol at 1 h) |

The results show that Alcalase CLEA is highly efficient under the conditions tested. 99% of Z-Ala-Phe-OMe was converted. When no additional buffer was added to the reaction mixture, the product contained approx. 91% Z-Ala-Phe-NH₂ and 8% free Z-Ala-Phe-OH. Addition of water increases ester hydrolysis yielding the free acid.

### Examples 19 and 20

### Effect of additional DMF on the amidation of a dipeptide when Alcalase CLEA is used

A stock solution of Z-Ala-Phe-OMe in DMF (200 mM) was prepared. 0.5 mmol of the ammonium source was added to 0.25 ml of the dipeptide stock solution. DMF and Bu*^{t}*OH was added corresponding to the ratio given in Table 5, for a total reaction volume of 5 ml. The reaction mixture was incubated at 30°C and the reaction was started by the addition of approximately 20 mg of Alcalase-CLEA. A sample (2 ml) was taken after two hours. 2 ml of acetonitrile was added and the mixture was centrifuged. The supernatants were analyzed by HPLC. The results are shown in Table 7 (initial conditions: 10 mM Z-Ala-Phe-OMe, 100 mM NH₂COONH₄, pH 7, 30°C).

**Table 7**

| **Ex.** | **H₂O/Bu*^{t}*OH/DMF** | **Z-Ala-Phe-NH₂ a/a%** | **Z-Ala-Phe-OH a/a%** | **Z-Ala-Phe-OMe a/a%** |
|---|---|---|---|---|
| 19 | 0/70/30 | 91.4 | 7.6 | 1.1 |
| 20 | 0/60/40 | 91 | 7.4 | 1.6 |

### Examples 21 to 30

### General procedure for screening different substrates with Alcalase CLEA and ammonium carbamate

In a typical experiment, the assay mixture contained approximately 10 mM substrate and 100 mM ammonium carbamate in an anhydrous solvent mixture of 17.5 % (vol/vol) DMF in Bu*^{t}*OH in a total reaction volume of 5 ml. The reaction mixture was thermostated at 30°C. The reaction was initiated by the addition of 4 mg/ml of Alcalase-CLEA. Reaction was incubated at 30 °C for 75 hours. Aliquot samples were taken at 2 h, 4 h and 75 h and the reaction was stopped by the addition of an equal volume of acetonitrile. Samples were analyzed by HPLC. All experiments were performed *in duplo.*

**Table 8**

| **Ex.** | **Peptide substrate** | **Reaction time (h)** | **P-NH₂** | **P-OH** | **P-OMe** |
|---|---|---|---|---|---|
| | | | **a/a%** | **a/a%** | **a/a%** |
| 21 | Z-Val-Phe-OMe | 2 | 77.0 | 16.4 | 6.7 |
| | | 4 | 82.1 | 17.2 | 6.7 |
| | | 75 | 86.7 | 13.4 | 0.0 |
| 22 | Z-Val-Tyr-OMe | 2 | 41.2 | 13.1 | 45.8 |
| | | 4 | 44.4 | 20.0 | 35.7 |
| | | 75 | 59.3 | 40.0 | 0.8 |
| 23 | Z-Val-Leu-OMe | 2 | 73.4 | 19.5 | 7.2 |
| | | 4 | 73.5 | 26.5 | 0.0 |
| 24 | Z-Val-Thr-OMe | 2 | 87.4 | 7.7 | 5.0 |
| | | 4 | 90.8 | 9.2 | 0.0 |
| 25 | Z-Val-Ala-OMe | 2 | 88.9 | 11.2 | 0.0 |
| 26 | Z-Val-Met-OMe | 2 | 71.1 | 28.1 | 0.9 |
| | | 4 | 72.8 | 27.3 | 0.0 |
| 27 | Z-Val-Lys(Boc)-OMe | 2 | 22.9 | 7.2 | 70.0 |
| | | 4 | 33.1 | 12.2 | 54.7 |
| | | 75 | 74.8 | 24.0 | 1.3 |
| 28 | Z-Ala-Phe-OMe | 2 | 91.7 | 6.7 | 1.7 |
| | | 4 | 92.9 | 6.7 | 0.5 |
| 29 | Z-Ala-Phe-Ala-OMe | 2 | 82.8 | 8.5 | 8.8 |
| | | 4 | 92.2 | 6.9 | 1.0 |
| 30 | Z-Ala-Phe-D-Ala-OMe | 2 | 6.0 | 0.3 | 93.7 |
| | | 4 | 10.8 | 0.8 | 88.5 |
| | | 75 | 35.4 | 10.4 | 54.3 |

With the exception of Z-Val-Tyr-OMe, Z-Val-Lys(Boc)-OMe and Z-Ala-Phe-D-Ala-OMe, all substrate peptides were quantitatively converted into products, *i.e*. the corresponding amide peptide and free carboxylic acid peptide, in 4h reaction time. At longer reaction times, also the methyl ester peptides ending with Tyr and Lys(Boc) were totally converted. Surprisingly, the methyl ester of the tripeptide with a C-terminal D-Ala residue was also converted. The HPLC analysis at 75 h reaction time showed conversion of 54 a/a% of substrate to yield about 36 a/a% of the product Z-Ala-Phe-D-Ala-NH₂.

### Examples 31 to 45 and Comparative Example I

### General procedure for screening the effect of different ammonium salts

In a typical experiment, the assay mixture contained approximately 10 mM peptide ester Z-Ala-Phe-OMe and 100 mM ammonium salt in an anhydrous solvent mixture of 17.5 % (vol/vol) DMF in Bu*^{t}*OH in a total reaction volume of 5 ml. The reaction mixture was thermostated at 30°C. The reaction was initiated by the addition of the enzyme (Alcalase-CLEA: 4 mg/ml or Subtilisin A (from SigmaAldrich): 1 mg/ml). Reaction was incubated at 30 °C for 21 hours. Aliquot samples were taken at 2 h, 4 h and 21 h and the reaction was stopped by the addition of an equal volume of acetonitrile. Samples were analyzed by HPLC. All experiments were performed *in duplo.* The results for Alcalase CLEA are shown in Table 9. The results for Subtilisin A are shown in Table 10.

**Table 9 (Alcalase CLEA)**

| **Ex.** | **Ammonium salt** | **Reaction time h** | **Z-Ala-Phe-NH₂ a/a%** | **Z-Ala-Phe-OH a/a%** | **Z-Ala-Phe-OMe a/a%** |
|---|---|---|---|---|---|
| 31 | NH₂COONH₄ Carbamate | 2 | 92.3 | 7.7 | 0 |
| | | 4 | 92.3 | 7.7 | 0 |
| | | 21 | 91.4 | 8.6 | 0 |
| 32 | CH₃COONH₄ Acetate | 2 | 83.4 | 12.5 | 4.1 |
| | | 4 | 87.2 | 12.3 | 0.5 |
| | | 21 | 91.2 | 8.8 | 0 |
| 33 | (NH4)₂CO₃ Carbonate | 2 | 88.1 | 11.9 | 0.0 |
| | | 4 | 88.8 | 11.2 | 0.0 |
| | | 21 | 90.8 | 9.2 | 0 |
| 34 | NH₄HCO₃ Bicarbonate | 2 | 84.7 | 14.9 | 0.4 |
| | | 4 | 85.7 | 14.3 | 0 |
| | | 21 | 88.3 | 11.7 | 0 |
| 35 | C₆H₅COONH₄ Benzoate | 2 | 61.1 | 30.4 | 8.5 |
| | | 4 | 68.7 | 29.8 | 1.5 |
| | | 21 | 87.7 | 12.3 | 0 |
| 36 | HCOONH₄ 4 | 2 | 31.6 | 19.8 | 48.5 |
| | | | 41.9 | 26.6 | 31.5 |
| | | 21 | 69.5 | 30.5 | 0 |
| 37 | NH₄F Fluoride | 2 | 26.9 | 15.1 | 57.9 |
| | | 4 | 41.2 | 20.5 | 38.2 |
| | | 21 | 71.0 | 27.0 | 1.9 |
| 38 | (NH₄)₃C₆H₅O₇ Citrate | 2 | 5.6 | 47.9 | 46.4 |
| | | 4 | 12.6 | 61.0 | 26.4 |
| | | 21 | 77.6 | 22.4 | 0 |
| 39 | (NH₄)₂C₂O₄.H₂O Oxalate monohydrate | 2 | 3.7 | 48.2 | 48.1 |
| | | 4 | 10.5 | 62.9 | 26.6 |
| | | 21 | 68.1 | 31.9 | 0 |
| 40 | (NH₄)₂C₄H₄O₆ Tartrate | 2 | 6.3 | 37.7 | 55.9 |
| | | 4 | 17.6 | 50.8 | 31.6 |
| | | 21 | 68.2 | 31.8 | 0 |
| 41 | (NH₄)₂SO₃.H₂O Sulphite hydrate | 2 | 2.9 | 6.1 | 91.0 |
| | | 4 | 7.2 | 12.8 | 80.0 |
| | | 21 | 60.7 | 39.3 | 0 |
| I | NH₄Cl Chloride | 2 | 6.8 | 38.8 | 54.4 |
| | | 4 | 14.8 | 52.9 | 32.3 |
| | | 21 | 68.5 | 31.5 | 0 |

Ammonium carbamate, ammonium acetate, ammonium carbonate, ammonium bicarbonate, and ammonium benzoate show excellent performance.

**Table 10 (Subtilisin A)**

| **Ex.** | **Ammonium source** | **Reaction time, h** | **Z-Ala-Phe-NH₂ a/a%** | **Z-Ala-Phe-OH a/a%** | **Z-Ala-Phe-OMe a/a%** |
|---|---|---|---|---|---|
| 42 | NH₂COONH₄ Carbamate | 2 | 36.9 | 4.8 | 48.4 |
| | | 4 | 60.2 | 8.2 | 31.6 |
| | | 21 | 86.8 | 12.9 | 0.3 |
| 43 | CH₃COONH₄ Acetate | 2 | 42.4 | 7.9 | 49.7 |
| | | 4 | 58.6 | 11.6 | 29.8 |
| | | 21 | 82.1 | 17.1 | 0.8 |
| 44 | NH₄HCO₃ Bicarbonate | 2 | 27.2 | 9.8 | 62.9 |
| | | 4 | 46.6 | 16.1 | 37.3 |
| | | 21 | 76.0 | 23.7 | 0.3 |
| 45 | (NH₄)₂CO₃ Carbonate | 2 | 42.4 | 7.9 | 49.7 |
| | | 4 | 53.1 | 12.0 | 34.9 |
| | | 21 | 80.9 | 8.7 | 0.4 |

Table 10 shows the results of the conversion of Z-Ala-Phe-OMe into amide with Subtilisin A, with ammonium carbamate, ammonium acetate, ammonium bicarbonate and ammonium carbonate as source of ammonia. High substrate conversion is obtained after 21 h reaction for the selected salts. Highest yield (i.e. 86 %) of the amide Z-Ala-Phe-NH₂ was obtained for ammonium carbamate at a reaction time of 21 h. More product of the ester hydrolysis is obtained for free Subtilisin A as compared to Alcalase-CLEA.

### Examples 46 to 53

### General procedure for screening different substrates with Subtilisin A and ammonium carbamate

In a typical experiment, the assay mixture contained approximately 10 mM peptide ester and 100 mM ammonium carbamate in an anhydrous solvent mixture of 17.5 % (vol/vol) DMF in Bu*^{t}*OH in a total reaction volume of 5 ml. The reaction mixture was thermostated at 30°C. The reaction was initiated by the addition of 1 mg/ml of Subtilisin A (from Sigma Aldrich). Reaction was incubated at 30 °C for 21 hours. Aliquot samples were taken at 2 h, 4 h and 21 h and the reaction was stopped by the addition of an equal volume of acetonitrile. Samples were analyzed by HPLC. All experiments were performed in duplicate. Results are given in Table 11.

**Table 11**

| **Ex.** | **Peptide substrate** | **Reaction time, h** | **P-NH₂ a/a%** | **P-OH a/a%** | **P-OMe a/a%** |
|---|---|---|---|---|---|
| 46 | Z-Val-Phe-OMe | 2 | 14.8 | 5.6 | 79.6 |
| | | 4 | 26.9 | 10.7 | 62.4 |
| | | 21 | 61.0 | 29.1 | 9.9 |
| 47 | Z-Val-Tyr-OMe | 2 | 5.8 | 3.0 | 91.2 |
| | | 4 | 11.1 | 5.6 | 83.3 |
| | | 21 | 29.6 | 18.3 | 52.1 |
| 48 | Z-Val-Leu-OMe | 2 | 14.3 | 6.9 | 78.8 |
| | | 4 | 25.9 | 12.1 | 62.0 |
| | | 21 | 58.0 | 33.7 | 8.3 |
| 49 | Z-Val-Thr-OMe | 2 | 18.2 | 2.3 | 79.4 |
| | | 4 | 34.4 | 4.5 | 61.1 |
| | | 21 | 76.7 | 13.7 | 9.6 |
| 50 | Z-Val-Ala-OMe | 2 | 50.1 | 10.0 | 39.9 |
| | | 4 | 70.0 | 14.4 | 15.6 |
| | | 21 | 82.1 | 17.9 | 0.0 |
| 51 | Z-Val-Met-OMe | 2 | 27.5 | 7.8 | 64.7 |
| | | 4 | 42.3 | 15.6 | 42.2 |
| | | 21 | 55.7 | 44.3 | 0.0 |
| 52 | Z-Val-Lys(Boc)-OMe | 2 | 1.6 | 0.0 | 98.4 |
| | | 4 | 2.9 | 1.2 | 95.9 |
| | | 21 | 10.5 | 6.7 | 82.7 |
| 53 | Z-Ala-Phe-OMe | 2 | 36.9 | 4.8 | 58.4 |
| | | 4 | 60.2 | 8.2 | 31.6 |
| | | 21 | 86.8 | 12.9 | 0.3 |

The specificity of Alcalase CLEA for various terminal amino acids was reported in Examples 21 to 30. Here, the results are reported obtained for testing the substrate specificity of soluble subtilisin. All peptide substrates were transformed into amides, but with lower amide yields than in the case of Alcalase-CLEA. Best substrates were Z-Ala-Phe-OMe (86.8 % amide after 21 h) and Z-Val-Ala-OMe (82 % amide formed after 21 h), while Z-Val-Lys(Boc)-OMe was the worse substrate. Extensive ester hydrolysis was observed. It appears that Alcalase CLEA and Subtilisin A have slightly different substrate specificity.

### Examples 54 to 68

### General procedure for screening different polar organic solvents

In a typical experiment, the assay mixture contained approximately 10 mM Z-Ala-Phe-OMe and 100 mM ammonium carbamate in an anhydrous solvent mixture containing 82.5 % dry Bu*^{t}*OH or Am*^{t}*OH and 17.5 % (vol/vol) of a co-solvent. The total reaction volume was 5 ml. The reaction mixture was thermostated at 30°C. The reaction was initiated by the addition of the enzyme (Alcalase-CLEA: 4 mg/ml or Subtilisin A (Sigma Aldrich): 1 mg/ml). Reaction was incubated at 30 °C for 22 hours. Aliquot samples were taken at 2 h, 4 h and 22 h and the reaction was stopped by the addition of an equal volume of acetonitrile. Samples were analyzed by HPLC. All experiments were performed in duplicate. The results for Alcalase CLEA are shown in Table 12. The results for Subtilisin A are shown in Table 13.

**Table 12 (Alcalase CLEA)**

| **Ex.** | **Solvent (82.5:17.5 (% vol/vol))** | **Reaction time h** | **Z-Ala-Phe-NH₂ a/a%** | **Z-Ala-Phe-OH a/a%** | **Z-Ala-Phe-OMe a/a%** |
|---|---|---|---|---|---|
| 54 | Bu*^{t}*OH/DMF | 2 | 92.0 | 8.0 | 0.0 |
| | | 4 | 92.1 | 7.9 | 0.0 |
| | | 22 | 91.3 | 8.7 | 0.0 |
| 55 | Bu*^{t}*OH/NMP | 2 | 90.0 | 7.6 | 2.4 |
| | | 4 | 91.9 | 7.8 | 0.3 |
| | | 22 | 91.3 | 8.7 | 0.0 |
| 56 | Bu*^{t}*OH /dioxane | 2 | 88.5 | 8.2 | 3.2 |
| | | 4 | 91.3 | 8.2 | 0.4 |
| | | 22 | 92.2 | 7.8 | 0.0 |
| 57 | Bu*^{t}*OH/DMA | 2 | 92.0 | 7.6 | 0.5 |
| | | 4 | 92.2 | 7.8 | 0.0 |
| | | 22 | 90.3 | 9.7 | 0.0 |
| 58 | Bu*^{t}*OH/DCM | 2 | 87.1 | 10.3 | 2.6 |
| | | 4 | 89.1 | 10.6 | 0.3 |
| | | 22 | 91.7 | 8.3 | 0.0 |
| 59 | Bu*^{t}*OH/THF | 2 | 91.5 | 7.8 | 0.7 |
| | | 4 | 92.3 | 7.7 | 0.0 |
| | | 22 | 93.3 | 6.7 | 0.0 |
| 60 | Bu*^{t}*OH /CH₃CN | 2 | 91.5 | 8.5 | 0.0 |
| | | 4 | 91.7 | 8.3 | 0.0 |
| | | 22 | 91.5 | 8.2 | 0.0 |
| 61 | Bu*^{t}*OH/DCE | 2 | 89.7 | 9.3 | 1.0 |
| | | 4 | 90.4 | 9.6 | 0.0 |
| | | 22 | 89.0 | 10.6 | 0.3 |
| 62 | Am*^{t}*OH/DMF* | 2 | 78.0 | 4.4 | 17.7 |
| | | 4 | 89.9 | 5.5 | 4.6 |
| | | 22 | 94 | 6 | 0 |
| 63 | Am*^{t}*OH/NMP* | 2 | 48.1 | 2.8 | 49.0 |
| | | 4 | 65.8 | 4.5 | 29.7 |
| | | 22 | 90.4 | 8.7 | 0.9 |

| | | | | | |
|---|---|---|---|---|---|
| * Alcalase CLEA concentration: 1 mg enzyme / ml reaction mixture. In all other experiments, the enzyme was used in concentration of 4 mg / ml. | | | | | |

**Table 13 (Subtilisin A)**

| **Ex.** | **Solvent (82.5:17.5 (% vol/vol))** | **Reaction time h** | **Z-Ala-Phe-NH₂ a/a%** | **Z-Ala-Phe-OH a/a%** | **Z-Ala-Phe-OMe a/a%** |
|---|---|---|---|---|---|
| 64 | Bu*^{t}*OH /DMF | 2 | 36.9 | 4.8 | 58.4 |
| | | 4 | 60.2 | 8.2 | 31.6 |
| | | 21 | 86.8 | 12.9 | 0.3 |
| 65 | Bu*^{t}*OH /NMP | 2 | 25.1 | 3.4 | 71.5 |
| | | 4 | 43 | 6 | 51 |
| | | 21 | 83.3 | 13.5 | 3.2 |
| 66 | Bu*^{t}*OH /dioxane | 2 | 9.9 | 1.4 | 88.6 |
| | | 4 | 16.3 | 2.4 | 81.3 |
| | | 21 | 44.6 | 7.5 | 47.9 |
| 67 | Bu*^{t}*OH /THF | 2 | 23.4 | 3.3 | 73.3 |
| | | 4 | 36 | 5.2 | 58.8 |
| | | 21 | 70.3 | 12.1 | 17.6 |
| 68 | Am*^{t}*OH /DMF | 2 | 22.2 | 3.5 | 74.3 |
| | | 4 | 36.9 | 6.1 | 57 |
| | | 21 | 73.4 | 21.1 | 5.4 |

Table 12 shows the results obtained for screening solvent effects when using Alcalase CLEA as catalyst. After 2 hours incubation, in experiments containing solvent mixtures of 82.5 Bu*^{t}*OH / 17.5 X (%vol/vol), when high amount of Alcalase CLEA was used (i.e. 4 mg enzyme / ml reaction mixture) total substrate conversion was obtained for all solvent combination used. The reaction mixture contained more than 87 % amide, and the amide yield increased above 93 % at longer reaction time.

Am*^{t}*OH proves to be an excellent solvent for the amidation of peptide methyl esters. When mixtures of Am*^{t}*OH /DMF (82.5 / 17.5 vol/vol) were used as solvent mixture, even at a lower enzyme concentration (Alcalase CLEA added approximately 1 mg enzyme / ml reaction mixture), high yields of amide (i.e. about 80 % amide) were obtained after 2 hours of incubation. At longer reaction time, at total substrate conversion, the product mixture contained more than 90 % amide. This suggests that Am*^{t}*OH can replace the Bu*^{t}*OH as main co-solvent in the reaction mixture for enzymatic amidation of methyl esters of peptides.

Similar trends were obtained when Subtilisin A was used (Table 13). However, Subtilisin A performs less than Alcalase CLEA in amide synthesis. About 85 % amide is obtained after 21 hours in 82.5 Bu*^{t}*OH / 17.5 DMF (% vol/vol). Generally, the amount of free peptide resulted from ester hydrolysis is higher, around 12-15 %. Binary solvent mixtures containing 82.5 % Bu*^{t}*OH or Am*^{t}*OH in combination with 17.5 % of either DMF and NMP seem to be the best solvent mixtures for Subtilisin A in amide synthesis.

### Examples 69 to 82

Since Am*^{t}*OH seemed to be a very good co-solvent in combination with DMF and NMP for the enzymatic amidation reaction as shown in Examples 55 to 69, these solvent mixtures were tested with the same procedure for some model reactions with Alcalase CLEA and Subtilisin A, for different peptide substrates and ammonium salts, respectively. Results are given in Table 14 for Alcalase CLEA (1 mg/ml) and Table 15 for Subtilisin A (0.2 mg/ml). The conclusion of these experiments is that Am*^{t}*OH can be used to replace Bu*^{t}*OH as cosolvent.

**Table 14 (Alcalase CLEA)**

| **Ex.** | **Reaction time, h** | **Ammonium salt** | **Peptide Substrate** | **Composition of reaction mixture** | | | |
|---|---|---|---|---|---|---|---|
| | | | | **Solvent (82.5:17.5)** | **% P-NH₂** | **% P-OH** | **% P-OMe** |
| 69 | 2 | NH₂COONH₄ | Z-Ala-Phe-OMe | Am*^{t}*OH/DMF | 78.0 | 4.4 | 17.7 |
| | 4 | | | | 89.9 | 5.5 | 4.6 |
| | 21 | | | | 94.0 | 6.0 | 0.0 |
| 70 | 2 | NH₂COONH₄ | Z-Val-Thr-OMe | Am*^{t}*OH /DMF | 47.9 | 2.8 | 49.3 |
| | 4 | | | | 64.8 | 4.6 | 30.6 |
| | 21 | | | | 80.3 | 14.6 | 5.1 |
| 71 | 2 | NH₂COONH₄ | Z-Val-Ala-OMe | Am*^{t}*OH /DMF | 78.5 | 8.6 | 12.9 |
| | 4 | | | | 86.2 | 9.9 | 4.0 |
| | 21 | | | | 87.6 | 10.6 | 1.8 |
| 72 | 2 | NH₂COONH₄ | Z-Ala-Phe-OMe | Am*^{t}*OH /NMP | 48.1 | 2.8 | 49.0 |
| | 4 | | | | 65.8 | 4.5 | 29.7 |
| | 21 | | | | 90.4 | 8.7 | 0.9 |
| 73 | 2 | NH₂COONH₄ | Z-Val-Thr-OMe | Am*^{t}*OH /NMP | 39.4 | 2.0 | 58.5 |
| | 4 | | | | 57.1 | 3.7 | 39.3 |
| | 21 | | | | 85.7 | 10.4 | 3.9 |
| 74 | 2 | NH₂COONH₄ | Z-Val-Ala-OMe | Am*^{t}*OH /NMP | 60.1 | 7.7 | 32.2 |
| | 4 | | | | 74.7 | 10.3 | 14.9 |
| | 21 | | | | 85.3 | 13.1 | 1.6 |
| 75 | 2 | NH₂COONH₄ | Z-Ala-Phe-OMe | Am*^{t}*OH /DMF | 78 | 4.4 | 17.7 |
| | 4 | | | | 89.9 | 5.5 | 4.6 |
| | 21 | | | | 94 | 6 | 0 |
| 76 | 2 | (NH₄)₂CO₃ | Z-Ala-Phe-OMe | Am*^{t}*OH /DMF | 68.7 | 6.1 | 25.2 |
| | 4 | | | | 83.5 | 8.1 | 8.3 |
| | 21 | | | | 90.9 | 9.1 | 0 |
| 77 | 2 | CH₃COONH₄ | Z-Ala-Phe-OMe | Am*^{t}*OH /DMF | 39 | 2.6 | 58.4 |
| | 4 | | | | 52.8 | 3.9 | 43.3 |
| | 21 | | | | 86 | 9.9 | 4.1 |

**Table 15 (Subtilisin A)**

| **Ex.** | **Reaction time h** | **Ammonium salt** | **Peptide Substrate** | **Composition of reaction mixture** | | | |
|---|---|---|---|---|---|---|---|
| | | | | **Solvent (82.5:17.5)** | **% P-NH₂** | **% P-OH** | **% P-OMe** |
| 78 | 2 | NH₂COONH₄ | Z-Val-Thr-OMe | Am*^{t}*OH /DMF | 10.9 | 1.9 | 87.3 |
| | 4 | | | | 21.2 | 3.4 | 75.4 |
| | 21 | | | | 57.0 | 16.5 | 26.5 |
| 79 | 2 | NH₂COONH₄ | Z-Val-Ala-OMe | Am*^{t}*OH /DMF | 31.0 | 8.7 | 60.3 |
| | 4 | | | | 49.7 | 14.1 | 36.2 |
| | 21 | | | | 73.6 | 24.2 | 2.2 |
| 80 | 2 | NH₂COONH₄ | Z-Ala Phe-OMe | Am*^{t}*OH /DMF | 22.2 | 3.5 | 74.3 |
| | 4 | | | | 36.9 | 6.1 | 57.0 |
| | 21 | | | | 73.4 | 21.1 | 5.4 |
| 81 | 2 | (NH₄)₂CO₃ | Z-Ala Phe-OMe | Am*^{t}*OH /DMF | 24.9 | 6.5 | 68.6 |
| | 4 | | | | 32.0 | 9.2 | 58.8 |
| | 21 | | | | 69.1 | 26.5 | 4.4 |
| 82 | 2 | CH₃COONH₄ | Z-Ala Phe-OMe | Am*^{t}*OH /DMF | 30.3 | 5.8 | 63.9 |
| | 4 | | | | 45.2 | 9.7 | 45.1 |
| | 21 | | | | 73.7 | 21.0 | 5.3 |

### Examples 83 to 91

### Optimizing the amount of Alcalase CLEA in the reaction mixture

Previous experiments showed that Alcalase CLEA is very efficient in the amide synthesis. Experiments were performed to determine the lowest enzyme concentration that can be used in the reaction, to obtain high product yield in a reasonable reaction time.

In a typical experiment, the assay mixture contained approximately 10 mM peptide substrate and 100 mM ammonium carbamate in an anhydrous solvent mixture of 82.5 % Bu*^{t}*OH and 17.5 % (vol/vol) of DMF, in a total reaction volume of 5 ml. The reaction mixture was thermostated at 30°C. The reaction was initiated by the addition of the enzyme (Alcalase-CLEA: 0.2 mg/ml, 1 mg/ml and 2 mg/ml, respectively). Reaction was incubated at 30 °C for 21 hours. Aliquot samples were taken at 2 h, 4 h and 21 h and the reaction was stopped by the addition of an equal volume of acetonitrile. Samples were analyzed by HPLC. All experiments were performed in duplicate. The results are listed in Table 16.

**Table 16.**

| **Ex.** | **Reaction time, h** | **Enzyme concentration, mg/ml** | **% P-NH₂** | % **P-OH** | **% P-OMe** |
|---|---|---|---|---|---|
| Z-Ala-Phe-OMe | | | | | |
| 83 | 2 | 0.2 | 33.5 | 1.2 | 65.3 |
| | 4 | 0.2 | 51.7 | 2.4 | 45.9 |
| | 21 | 0.2 | 85 | 6.4 | 8.6 |
| 84 | 2 | 1 | 80.4 | 3.4 | 16.2 |
| | 4 | 1 | 92.3 | 4.2 | 3.5 |
| | 21 | 1 | 95.1 | 4.9 | 0 |
| 85 | 2 | 2 | 91.1 | 5.4 | 3.6 |
| | 4 | 2 | 94.5 | 5.5 | 0 |
| | 21 | 2 | 94 | 6 | 0 |

| Z-Val-Phe-OMe | | | | | |
|---|---|---|---|---|---|
| 86 | 2 | 0.2 | 14.5 | 1.3 | 84.1 |
| | 4 | 0.2 | 24.2 | 2.5 | 73.3 |
| | 21 | 0.2 | 55.7 | 11.4 | 32.9 |
| 87 | 2 | 1 | 49.8 | 5.2 | 44.9 |
| | 4 | 1 | 67.8 | 7.6 | 24.6 |
| | 21 | 1 | 87.8 | 11.8 | 0.4 |
| 88 | 2 | 2 | 69.5 | 9.7 | 20.8 |
| | 4 | 2 | 81.7 | 11.7 | 6.6 |
| | 21 | 2 | 88.4 | 11.6 | 0.0 |

| Z-Val-Thr-OMe | | | | | |
|---|---|---|---|---|---|
| 89 | 2 | 0.2 | 56.7 | 2.6 | 40.7 |
| | 4 | 0.2 | 76.4 | 3.9 | 19.7 |
| | 21 | 0.2 | 93.5 | 6.5 | 0.0 |
| 90 | 2 | 1.2 | 76.4 | 3.9 | 19.7 |
| | 4 | 1.2 | 76.4 | 3.9 | 19.7 |
| | 21 | 1.2 | 93.5 | 6.5 | 0.0 |
| 91 | 2 | 2 | 68.8 | 4.2 | 27.0 |
| | 4 | 2 | 84.1 | 5.8 | 10.0 |
| | 21 | 2 | 90.3 | 9.7 | 0.0 |

It can be seen that at an Alcalase CLEA concentration of 1 mg / ml reaction mixture, 95 % conversion of the substrate Z-Ala-Phe-OMe is obtained after 4 hours incubation. The product mixture contained 92 % amide and only 3.5 % of the secondary product of the ester hydrolysis. High amide yields were obtained for other peptide substrates, for this low enzyme concentration, but at longer reaction times. However, these results show that the concentration of enzyme, i.e. Alacalse-CLEA, can be reduced significantly, thus making the process more economical.

### Example 92

### Amidation of Boc-Pro-Pro-Ala-Phe-Ala-OMe

In a typical experiment, the assay mixture contained approximately 10 mM peptide substrate and 100 mM ammonium carbamate in an anhydrous solvent mixture of 82.5 % Bu*^{t}*OH and 17.5 % (vol/vol) of DMF, in a total reaction volume of 5 ml. The reaction mixture was thermostated at 30°C. The reaction was initiated by the addition of 4 mg/ml Alcalase CLEA. Reaction was incubated at 30 °C for 21 hours. Aliquot samples were taken at 2 h, 4 h and 21 h and the reaction was stopped by the addition of an equal volume of acetonitrile. Samples were analyzed by HPLC. The results are listed in Table 17.

**Table 17**

| **Ex.** | **Reaction time, h** | **% P-NH₂** | **% P-OH** | **% P-OMe** |
|---|---|---|---|---|
| 92 | 2 | 42.15 | 0.3 | 57.55 |
| | 4 | 60.73 | 0.55 | 38.72 |
| | 21 | 99.21 | - | 0.79 |

### Example 93

### Enzymatic amidation of Z-Ala-Phe-OH

In a typical experiment, the assay mixture contained approximately 10 mM peptide substrate with a C-terminal free acid and 100 mM ammonium carbamate in an anhydrous solvent mixture of 82.5 % Bu*^{t}*OH and 17.5 % (vol/vol) of DMF, in a total reaction volume of 5 ml. The reaction mixture was thermostated at 30°C. The reaction was initiated by the addition of 4 mg/ml Alcalase CLEA. Reaction was incubated at 30 °C for 21 hours. Aliquot samples were taken at 2 h, 4 h and 21 h and the reaction was stopped by the addition of an equal volume of acetonitrile. Samples were analyzed by HPLC. The results are listed in Table 18.

**Table 18**

| **Ex.** | **Reaction time, h** | **Z-Ala-Phe-NH₂ a/a%** | **Z-Ala-Phe-OH a/a%** |
|---|---|---|---|
| 93 | 2 | 29.14 | 70.86 |
| | 4 | 34.43 | 65.57 |
| | 21 | 69.86 | 30.14 |

It can be concluded from these experiments that the C-terminal free acid of a peptide substrate can also be amidated by ammonium carbamate in the presence of Alcalase CLEA.

## Claims

1. A process for the amidation of C-terminal esters or acids of peptide substrates in solution-phase synthesis of peptides, comprising amidating one or more peptide substrates comprising C-terminal esters or acids using the protease subtilisin in any suitable form in the presence of one or more ammonium salts derived from an acid having a pKa above 0.

2. A process according to claim 1 wherein C-terminal esters of peptide substrates are amidated.

3. A process according to claim 2 wherein the esters of the C-terminal esters of the peptide substrate are selected from the group of C₁₋₁₂ (ar)alkyl esters.

4. A process according to claim 3 wherein the esters of the C-terminal esters of the peptide substrate are selected from the group of primary C₁₋₁₂ (ar)alkyl esters.

5. A process according to claim 4 wherein the esters of the C-terminal esters of the peptide substrate are selected from the group of primary C₁₋₄ alkyl esters.

6. A process according to claim 5 wherein the ester of the C-terminal ester of the peptide substrate is the C-terminal methyl ester.

7. A process according to any one of the preceding claims wherein the ammonium salt is derived from an acid having a pKa above 3.5.

8. A process according to claim 7 wherein the ammonium salt has the following chemical structure (I): 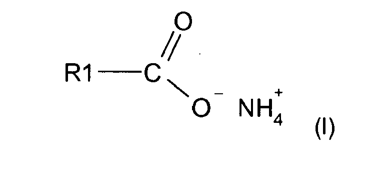 wherein
R1 is selected from the group of hydrogen, C₁₋₁₂ (ar)alkyl, C₆₋₁₂ aryl, -N(R2)₂, -OH, and R3-O⁻ NH₄⁺,
R2 is selected from the group of hydrogen and/or C₁₋₄ alkyl, and
R3 is a bond, a carbonyl group, or a C₁₋₄ alkyl carbonyl group, optionally substituted with one or more hydroxyl groups and/ or -COO⁻ NH₄⁺,
optionally in hydrated form.

9. A process according to claim 8 wherein R1 is selected from the group of C₁₋₁₂ (ar)alkyl, C₆₋₁₂ aryl, -NH₂, -OH, and -O⁻ NH₄⁺.

10. A process according to claim 9 wherein the ammonium salt is selected from ammonium carbamate, ammonium carbonate, ammonium bicarbonate, ammonium acetate, ammonium benzoate, and mixtures thereof.

11. A process according to claim 10 wherein the ammonium salt is ammonium carbamate.

12. A process according to any one of the preceding claims wherein the molar ratio of ammonium salt to peptide substrate ranges from 2:1 to 20:1.

13. The process according to any one of the preceding claims wherein the peptide substrate comprising the C-terminal ester or acid comprises a C-terminal acyl residue which is an α-amino acyl residue from natural or synthetic origin.

14. The process according to claim 13, wherein the α-amino acyl residue is selected from Ala, protected Cys, protected Asp, protected Glu, Phe, Gly, His, (protected) Lys, Leu, Met, Asn, Gln, (protected) Arg, (protected) Ser, Thr, Val, (protected) Trp and (protected) Tyr.

15. The process according to any one of the preceding claims wherein the peptide substrate is prepared according to a process for rapid solution synthesis of a peptide in an organic solvent or a mixture of organic solvents, the process comprising repetitive cycles of steps (a)-(d):
a) a coupling step, using an excess of an activated carboxylic component to acylate an amino component,
b) a quenching step in which a scavenger is used to remove residual activated carboxylic functions, wherein the scavenger may also be used for deprotection of the growing peptide,
c) one or more aqueous extractions and
optionally, (d) a separate deprotection step, followed by one or more aqueous extractions,
whereby in at least one cycle in process step b an amine comprising a free anion or a latent anion is used as a scavenger of residual activated carboxylic functions.

16. The process according to any one of the preceding claims wherein the protease subtilisin is of the family EC 3.4.21.62.

17. The process according to any one of the preceding claims wherein the protease subtilisin is free subtilisin.

18. The process according to any one of the preceding claims wherein the protease subtilisin is cross-linked enzyme aggregate (CLEA) subtilisin.

19. The process according to any one of the preceding claims wherein an organic solvent is used.

20. The process of claim 19, wherein the organic solvent is selected from *N*,*N-*dimethylformamide (DMF), N-methyl-2-pyrrolidone (NMP), dioxane, *N*,*N-*dimethylacetamide (DMA), dichloromethane (DCM), tetrahydrofuran (THF), acetonitrile, *tert*-butanol, *tert*-amyl alcohol, dichloroethane (DCE), *tert*-butyl methyl ether (MTBE), and mixtures thereof

21. The process of claim 20, wherein the organic solvent is a mixture of *tert*-butanol and DMF, *tert*-butanol and NMP, *tert*-amyl alcohol and DMF, or *tert*-amyl alcohol and NMP.

22. The process according to claims 19 to 21 wherein water is present in the organic solvent ranging from 0.0001 to 5% (v/v).

23. The process according to any one of the preceding claims wherein pH at which the reaction is performed is selected from the range of 5.5-10.

24. The process according to any one of the preceding claims wherein the reaction temperature for the amidation is 15-60°C.

25. The process according to any one of the preceding claims wherein the amount of enzyme ranges from 1 to 50 wt.% related to the peptide substrate.

26. The process according to any one of the preceding claims wherein the amidation is performed by stepwise adding portions of the protease subtilisin (in any suitable form) into the reaction mixture comprising one or more peptide substrates comprising C-terminal esters or acids.

27. The process according to any one of the preceding claims wherein the amidation is performed by stepwise adding portions of the ammonium salt into the reaction mixture comprising one or more peptide substrates comprising C-terminal esters or acids.

## Patentansprüche

1. Verfahren für die Amidierung von C-beendeten Estern oder Säuren von Peptidsubstraten in der Lösungsphasen-Synthese von Peptiden, umfassend das Amidieren von einem oder mehreren Peptidsubstraten, umfassend C-beendete Estern oder Säuren unter Einsatz von Protease-Subtilisin in jeglicher geeigneten Form in Gegenwart von einem oder mehreren Ammoniumsalzen, abgeleitet von einer Säure mit einem pKa grösser als 0.

2. Verfahren gemäss Anspruch 1, wobei C-beendete Ester von Peptidsubstraten amidiert sind.

3. Verfahren gemäss Anspruch 2, wobei die Ester der C-beendeten Ester des Peptidsubstrats ausgewählt sind aus der Gruppe of C₁₋₁₂(Ar)Alkyl-Ester.

4. Verfahren gemäss Anspruch 3, wobei die Ester der C-beendeten Ester des Peptidsubstrats ausgewählt sind aus der Gruppe von primären C₁₋₁₂(Ar)Alkylestern.

5. Verfahren gemäss Anspruch 4, wobei die Ester der C-beendeten Ester des Peptidsubstrats ausgewählt sind aus der Gruppe von primären C₁₋₄ Alkylestern.

6. Verfahren gemäss Anspruch 5, wobei der Ester der C-beendeten Ester des Peptidsubstrats der C-beendete Methylester ist.

7. Verfahren gemäss irgendeinem der vorstehenden Ansprüche, wobei das Ammoniumsalz von einer Säure mit einem pKa oberhalb von 3.5 abgeleitet ist.

8. Verfahren gemäss Anspruch 7, wobei das Ammoniumsalz die folgende chemische Struktur (I) hat: 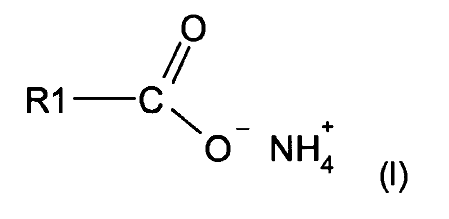 wobei
R1 ausgewählt ist aus der Gruppe aus Wasserstoff, C₁₋₁₂ (Ar)Alkyl, C₆₋₁₂ Aryl, -N(R2)₂, -OH, und R3-O⁻NH4⁺,
R2 ausgewählt ist aus der Gruppe aus Wasserstoff und/oder C₁₋₄ Alkyl, und
R3 eine Bindung, eine Carbonylgruppe, oder eine C₁₋₄ Alkyl carbonylgruppe ist, optional substituiert mit einem oder mehreren Hydroxylgruppen und/oder -COO⁻ NH₄⁺,
optional in hydrierter Form.

9. Verfahren gemäss Anspruch 8, wobei R1 ausgewählt ist aus der Gruppe aus C₁₋₁₂ (Ar)Alkyl, C₆₋₁₂ Aryl, -NH₂, -OH, und-O⁻ NH₄⁺.

10. Verfahren gemäss Anspruch 9, wobei das Ammoniumsalz ausgewählt ist aus Ammoniumcarbamat, Ammoniumcarbonat, Ammoniumbicarbonat, Ammoniumacetat, Ammoniumbenzoat, und Mischungen davon.

11. Verfahren gemäss Anspruch 10, wobei das Ammoniumsalz Ammoniumcarbamat ist.

12. Verfahren gemäss irgendeinem der vorstehenden Ansprüche, wobei das Molverhältnis vom Ammoniumsalz zu Peptidsubstrat zwischen 2:1 bis 20:1 beträgt.

13. Verfahren gemäss irgendeinem der vorstehenden Ansprüche, wobei das Peptidsubstrat umfassend den C-beendeten Ester oder Säure ein C-beendetes Acylresiduum aufweist, welches ein α-Aminoacylresiduum von natürlichem oder synthetischem Ursprung ist.

14. Verfahren gemäss Anspruch 13, wobei das α-Aminoacylresiduum ausgewählt ist aus Ala, geschütztem Cys, geschütztem Asp, geschütztem Glu, Phe, Gly, His, (geschütztem) Lys, Leu, Met, Asn, Gln, (geschütztem) Arg, (geschütztem) Ser, Thr, Val, (geschütztem) Trp und (geschütztem) Tyr.

15. Verfahren gemäss irgendeinem der vorstehenden Ansprüche, wobei das Peptidsubstrat gemäss dem Verfahren für schnelle Lösungssynthese eines Peptids in einem organischen Lösungsmittel oder einer Mischung von organischen Lösungsmitteln hergestellt ist, wobei das Verfahren sich wiederholende Zyklen von Schritten (a)-(d) umfasst:
a) einen Kopplungsschritt, einsetzend einen Überschuss einer aktivierten Carboxylkomponente, um eine Aminokomponente zu acylieren,
b) einen Quenchschritt, bei dem ein Radikalfänger eingesetzt wird, um residual aktivierte Carboxylfunktionen zu entfernen, wobei der Radikalfänger auch für das Entschützen des wachsenden Peptids eingesetzt werden kann,
c) eine oder mehrere wässrigen Extraktionen und
optional, d) einen getrennten Entschützungsschritt, gefolgt von einem oder mehreren wässrigen Extraktionen, wobei in mindestens einem Zyklus im Verfahrensschritt b ein Amin umfasssend ein freies Anion oder ein latentes Anion als ein Radikalfänger von residual aktivierten Carboxylfunkrionen eingesetzt wird.

16. Verfahren gemäss irgendeinem der vorstehenden Ansprüche, wobei das Proteasesubtilisin von der Familie EC 3.4.21.62 ist.

17. Verfahren gemäss irgendeinem der vorstehenden Ansprüche, wobei das Proteasesubtilisin freies Subtilisin ist.

18. Verfahren gemäss irgendeinem der vorstehenden Ansprüche, wobei das Proteasesubtilisin vernetztes Enzym aggregiertes (CLEA) Subtilisin ist.

19. Verfahren gemäss irgendeinem der vorstehenden Ansprüche, wobei ein organisches Lösungsmittel eingesetzt wird.

20. Verfahren gemäss Anspruch 19, wobei das organische Lösungsmittel ausgewählt ist aus *N*, *N*-Dimethylformamid (DMF), *N*-Methyl-2-pyrrolidon (NMP), Dioxan, N,N-Dimethylacetamid (DMA), Dichlormethan (DCM), Tetrahydrofuran (THF), Acetonitril, *tert*-Butanol, *tert*-Amylalkohol, Dichlorethan (DCE), *tert*-Butylmethylether (MTBE), und Mischungen davon.

21. Verfahren gemäss Anspruch 20, wobei das organische Lösungsmittel eine Mischung von *tert*-Butanol und DMF, *tert*-Butanol und NMP, *tert*-Amylalkohol und DMF, oder *tert*-Amylalkohol und NMP ist.

22. Verfahren gemäss Ansprüchen 19 bis 21, wobei Wasser in dem organischen Lösungsmittel in einem Bereich zwischen 0.0001 und 5% Volumenprozent vorhanden ist.

23. Verfahren gemäss irgendeinem der vorstehenden Ansprüche, wobei der pH-Wert, an dem die Reaktion ausgeführt wird, ausgewählt ist aus dem Bereich von 5.5-10.

24. Verfahren gemäss irgendeinem der vorstehenden Ansprüche, wobei die Reaktionstemperatur für die Amidierung zwischen 15-60° Celsius ist.

25. Verfahren gemäss irgendeinem der vorstehenden Ansprüche, wobei die Menge an Enzym zwischen 1 und 50 Gewichtsprozent in Bezug auf das Peptidsubstrat beträgt.

26. Verfahren gemäss irgendeinem der vorstehenden Ansprüche, wobei die Amidierung ausgeführt wird durch schrittweises Hinzufügen von Anteilen des Proteasesubtilisins (in jeglicher geeigneten Form) in die Reaktionsmischung umfassend eines oder mehrere Peptidsubstrate umfassend C-beendete Ester oder Säuren.

27. Verfahren gemäss irgendeinem der vorstehenden Ansprüche, wobei die Amidierung ausgeführt wird durch schrittweises Hinzufügen von Anteilen des Ammoniumsalzes in die Reaktionsmischung umfassend eines oder mehrere Peptidsubstrate umfassend C-beendete Ester oder Säuren.

## Revendications

1. Un procédé pour l'amidation d'esters ou acides C-terminaux de substrats peptidiques par synthèse en phase soluble de peptides, comprenant l'amidation de un ou plusieurs substrats peptidiques comprenant des esters ou acides C-terminaux par action de protéase subtilisine, sous n'importe quelle forme appropriée, en présence d'un ou plusieurs sels d'ammonium dérivés d'un acide ayant un pKa supérieur à 0.

2. Un procédé selon la revendication 1, dans lequel les esters C-terminaux de substrats peptidiques sont amidés.

3. Un procédé selon la revendication 2, dans lequel les esters des esters C-terminaux du substrat peptidique sont choisis dans le groupe des esters d'(ar)alkyl en C₁₋₁₂.

4. Un procédé selon la revendication 3, dans lequel les esters des esters C-terminaux du substrat peptidique sont choisis dans le groupe des esters d'(ar)alkyl en C₁₋₁₂ primaires.

5. Un procédé selon la revendication 4, dans lequel les esters des esters C-terminaux du substrat peptidique sont choisis dans le groupe des esters d'alkyl en C₁₋₄ primaires.

6. Un procédé selon la revendication 5, dans lequel l'ester de l'ester C-terminal du substrat peptidique est le méthyl ester C-terminal.

7. Un procédé selon une quelconque des revendications précédentes, dans lequel le sel d'ammonium dérive d'un acide ayant un pKa supérieur à 3.5.

8. Un procédé selon la revendication 7, dans lequel le sel d'ammonium présente la structure chimique suivante (I): 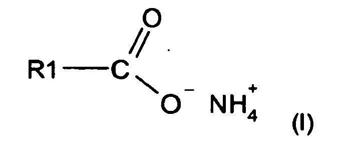 dans laquelle
- R1 est choisi dans le groupe comprenant hydrogène, (ar)alkyl en C₁₋₁₂, aryl en C₆₋₁₂, -N(R2)₂, -OH, et R3-O⁻NH₄⁺,
- R2 est choisi dans le groupe comprenant hydrogène et/ou alkyl en C₁₋₄, et
- R3 est une liaison, un groupe carbonyl, ou un groupe (alkyl en C₁₋₄)-carbonyl, éventuellement substitués par un ou plusieurs groupes hydroxyl et/ ou -COONH₄⁺
éventuellement sous forme hydraté.

9. Un procédé selon la revendication 8, dans lequel R1 est choisi dans le groupe (ar)alkyl en C₁₋₁₂, aryl en C₆₋₁₂, -NH₂, -OH, et -O⁻NH₄⁺.

10. Un procédé selon la revendication 9, dans lequel le sel d'ammonium est choisi parmi le carbamate d'ammonium, le carbonate d'ammonium, le bicarbonate d'ammonium, l'acétate d'ammonium, le benzoate d'ammonium et leurs mélanges.

11. Un procédé selon la revendication 10, dans lequel le sel d'ammonium est le carbamate d'ammonium.

12. Un procédé selon une quelconque des revendications précédentes, dans lequel le rapport molaire sel d'ammonium/substrat peptidique est compris entre 2/1 et 20/1.

13. Le procédé selon une quelconque des revendications précédentes, dans lequel le substrat peptidique comprenant l'ester ou acide C-terminal comprend un résidu acyl C-terminal qui consiste en un résidu α-amino acyl d'origine naturelle ou synthétique.

14. Le procédé selon la revendication 13, dans lequel le résidu α-amino acyl est choisi parmi Ala, Cys protégé, Asp protégé, Glu protégé, Phe, Gly, His, Lys (protégé), Leu, Met, Asn, Gln, Arg (protégé), Ser (protégé), Thr, Val, Trp (protégé) et Tyr (protégé).

15. Le procédé selon une quelconque des revendications précédentes, dans lequel le substrat peptidique est préparé selon un procédé de synthèse en solution rapide d'un peptide dans un solvant organique ou un mélange de solvants organiques, le procédé comprenant des cycles répétitifs des étapes (a) à (d):
(a) une étape de couplage, faisant emploi d'un excès d'un dérivé carboxylique activé pour acyler un dérivé amino,
(b) une étape d'atténuation ("quenching") dans laquelle est employé un scavenger pour éliminer les fonctions carboxyliques activées résiduelles, ce scavenger pouvant également être utilisé pour la déprotection du peptide en croissance,
(c) une ou plusieurs extractions aqueuses et
éventuellement,
(d) une étape de déprotection distincte, suivie par une ou plusieurs extractions aqueuses,
où dans au moins un des cycles de l'étape (b) du procédé est employée une amine comprenant un anion libre ou un anion latent agissant en tant que scavenger à l'égard des fonctions carboxyliques activées résiduelles.

16. Le procédé selon une quelconque des revendications précédentes, dans lequel la protéase subtilisine appartient à la famille EC 3.4.21.62.

17. Le procédé selon une quelconque des revendications précédentes, dans lequel la protéase subtilisine est de la subtilisine libre.

18. Le procédé selon une quelconque des revendications précédentes, dans lequel la protéase subtilisine est de la subtilisine d'agrégat d'enzyme réticulée (CLEA).

19. Le procédé selon une quelconque des revendications précédentes, dans lequel est utilisé un solvant organique.

20. Le procédé selon la revendication 19, dans lequel le solvant organique est choisi parmi *N*,*N-*diméthylformamide (DMF), N-méthyl-2-pyrrolidone (NMP), dioxane, *N*,*N-*diméthylacétamide (DMA), dichlorométhane (DCM), tétrahydrofuranne (THF), acétonitrile, *tert*-butanol, alcool *tert*-amylique, dichloroéthane (DCE), *tert*-butyl méthyl éther (MTBE), et leurs mélanges.

21. Le procédé de la revendication 20, dans lequel le solvant organique est un mélange de *tert*-butanol et de DMF, de *tert*-butanol et de NMP, d'alcool *tert-*amylique et de DMF, ou d'alcool *tert*-amylique et de NMP.

22. Le procédé selon les revendications 19 à 21, dans lequel l'eau est présente dans le solvant organique en une proportion allant de 0,0001 à 5% (v/v).

23. Le procédé selon une quelconque des revendications précédentes, dans lequel le pH auquel la réaction est mise en oeuvre est choisi dans la gamme comprise entre 5.5 et 10.

24. Le procédé selon une quelconque des revendications précédentes, dans lequel la température de réaction pour l'amidation est comprise entre 15-60°C.

25. Le procédé selon une quelconque des revendications précédentes, dans lequel la quantité d'enzyme est comprise entre 1 et 50% en poids exprimés par rapport au substrat peptidique.

26. Le procédé selon une quelconque des revendications précédentes, dans lequel l'amidation est mise en oeuvre par addition fractionnée de portions de protéase subtilisine (sous n'importe quelle forme appropriée) dans le mélange réactionnel comprenant un ou plusieurs substrats peptidiques comprenant des esters ou acides C-terminaux.

27. Le procédé selon une quelconque des revendications précédentes, dans lequel l'amidation est mise en oeuvre par addition fractionnée de portions de sel d'ammonium dans le mélange réactionnel comprenant un ou plusieurs substrats peptidiques comprenant des esters ou acides C-terminaux.
